**Europäisches Patentamt**

**European Patent Office**

**Office européen des brevets**

(19)

(11) Publication number : **0 322 446 B1**

(12)

# EUROPEAN PATENT SPECIFICATION

(45) Date of publication of patent specification :
**19.02.92 Bulletin 92/08**

(51) Int. Cl.⁵ : **G01N 29/04,** G01N 29/00

(21) Application number : **88906621.3**

(22) Date of filing : **11.05.88**

(86) International application number :
**PCT/US88/01557**

(87) International publication number :
**WO 88/09502 01.12.88 Gazette 88/26**

(54) BOND STRENGTH MEASUREMENT OF COMPOSITE PANEL PRODUCTS.

(30) Priority : **26.05.87 US 53909**
**06.04.88 US 172719**

(43) Date of publication of application :
**05.07.89 Bulletin 89/27**

(45) Publication of the grant of the patent :
**19.02.92 Bulletin 92/08**

(84) Designated Contracting States :
**BE DE FR GB SE**

(56) References cited :
**FR-A- 2 488 996**
**GB-A- 1 150 680**
**US-A- 3 315 520**
**ISA Transactions, vol 6, no. 4 1967, J.T.Collins:**
**" Detection of blisters and blows in plywood ",**
**pages 303-306. see the whole document.**

(73) Proprietor : **WEYERHAEUSER COMPANY**
**Tacoma, WA 98477 (US)**

(72) Inventor : **SHEARER, Dwayne, M.**
**4838 N.E. 43rd Street**
**Seattle, WA 98105 (US)**
Inventor : **BEETHAM, Richard, C.**
**31114 S.E. 353rd Street**
**Enumclaw, WA 98002 (US)**
Inventor : **BEALL, Frank, C.**
**2505 Manorwood Drive**
**Puyallup, WA 98371 (US)**
Inventor : **RODGERS, John, M.**
**4429 Edison Avenue**
**Sacramento, CA 95821 (US)**

(74) Representative : **Patentanwälte Grünecker,**
**Kinkeldey, Stockmair & Partner**
**Maximilianstrasse 58**
**W-8000 München 22 (DE)**

## Description

The present invention relates to methods for the on-line, nondestructive determination of a mechanical property of composite panel products of the type as outlined in the preambles of claims 1 and 10.

A method of this type is disclosed in FR-A-2 488 996. These methods are particularly useful as a process and quality control tool wherein it can give an immediate appraisal of mechanical properties of products such as wood fiber, particle, flake, or strand boards. Measurements made shortly after pressing products of this type correlate well with conventional laboratory methods made several hours or days later. In accordance with the method of FR-A-2 488 996 the thickness of the panels will be measured, and the transducer output signal will be representative for the retardment of the waves at the receiving transducer. From these values the flexural or tensile strength of the panels will be calculated.

For composite wood products such as those named above internal bond strength and flexural strength are probably the two most important physical indicators of board quality. Flexural strength, sometimes termed modulus of rupture, is most indicative of surface strength while internal bond is more indicative of the interior or core strength of the product. In the past, it has been a matter of hours, or even days, before the results of quality control tests would be available to production personnel. This often resulted in large quantities of products being made which were outside quality specifications with nobody being aware of the problems.

In recent years further equipment has been developed to attempt to nondestructively measure certain physical properties either on line during production or shortly thereafter. For example, Collins, in U.S. Patents 3 423 991 and 3 780 570 shows systems using rolling transducers useful for determining voids or delamination in plywood. One transducer directs a pulse of ultrasonic energy against one face of the panel. An opposing transducer analyses exponentially decaying reverberations to obtain the desired information. Other devices measure pulse propagation velocity as an indicator of the physical property being measured. One such device is shown in U.S. Patent 4 073 007 to Boivin. Here the time for a pulse to travel a predetermined distance is used as an indicator for measuring lack of planarity of a tensioned sheet metal strip. Logan, in U.S. Patent 4 201 093, uses a rolling oil filled transducer to introduce ultrasonic signals into sheets.

A common feature of the Collins and Logan devices is the use of a transducer element contained within a rolling wheel or cylinder. Various means are used to couple energy from the transducer to the wheel and from there into the material being measured.

Devices of the types just described have achieved only limited use for a number of reasons. In many cases the reliability is very poor. In other cases, the device is unable to measure the desired physical property with sufficient accuracy and/or reliability. A system described in U.S. Patent 4,036,057 to Morais has been used to determine adhesion or delamination in wood products and the tensile strength of graphite-epoxy composites. This uses an ultrasonic signal to propagate a stress wave through the material being tested. A receiving transducer detects the "ringdown" or decay of resonant waves produced within the substrate being tested. A device of the Morais type has been shown to give results having a general correlation with the internal bond strength of composite panel products such as particleboard. However, this correlation is so poor that is not usefully predictive for use as a quality control tool.

It will therefore be appreciated that there has been a significant need of a method which overcomes the problems experienced with prior art on-line testing devices and which is capable of the accurate determination of the internal bond strength of numerous types of composite panel products.

The present invention fulfills this need by a method for on-line, nondestructive determination of the internal bond strength of composite panel products according to claims 1 or 10. The first step in the method is to measure the thickness of the product. Where the panels are of relatively uniform thickness, they may be assigned to a thickness class; e.g., 12-13 mm, 19-20mm, etc. The consideration of thickness and temperature of the panels being measured has proved to be a critical and key improvement when used together with some of the prior ultrasonic techniques.

As the next step in the process, an ultrasound pulse is impinged against the panel through a first tranducer which may or may not be in contact with the panel. This transmitted pulse is received by a second transducer spaced apart from the first transducer. This also either may or may not be in contact with the panel. The signal received by the second transducer is expressed as an output voltage. Then the thickness, panel surface temperature, and output signal voltage are entered into an equation of the form

$$IB = a + b(\text{output voltage}) + c(\text{temperature}) + d(\text{thickness}) + e(\text{thickness})^2$$

The coefficients a, b, c, d and e can readily be determined experimentally for the particular equipment being used and product being tested. Finally, the equation is solved to give an estimate of the internal bond strength. This is most conveniently done using a dedicated microprocessor or computer which will give a visible readout or printout to a plant operator.

It will be readily evident that the particuar order of the steps described is not critical. Thus, temperature and panel thickness could be measured following the ultrasound procedure.

In many cases the accuracy of the method is sufficient when, as noted before, the panels are simply assigned to a thickness class. In this case the second order equation just described can be simplified to a first order equation with the variables being only the second transducer output voltage and the panel temperature. In this case the equation has the following form

$$IB = a + b(\text{output voltage}) + c(\text{temperature})$$

Here the coefficients a, b and c must be determined for each individual thickness class being tested.

It is an object of the present invention to provide a method for the accurate nondestructive determination of the internal bond of composite wood panels.

It is another object to provide a method for on-line determination of the internal bond of particleboard at or near the point of press discharge.

It is a further object to provide a method for on-line determination of internal bond in composite panels that is not subject to the inaccuracies inherent in related prior art methods.

These and many other objects will become readily apparent to those skilled in the art upon reading the following detailed description of preferred embodiments taken in conjunction with the drawings.

Figure 1 is a side elevation view of key mechanical components of the testing apparatus.

Figure 2 shows the interior detail of a suitable rotary transducer.

Figure 3 is an end elevation view showing the arrangement of the upper and lower transducers.

Figure 4 is a graph showing the measurement of ringdown count.

Figure 5 is a graph showing the predicted internal bond from on-line measurement compared with internal bond of the same panels measured in the laboratory.

Figures 6 and 7 are end elevation views, similar to Figure 3, showing alternative transducer type and positioning arrangements.

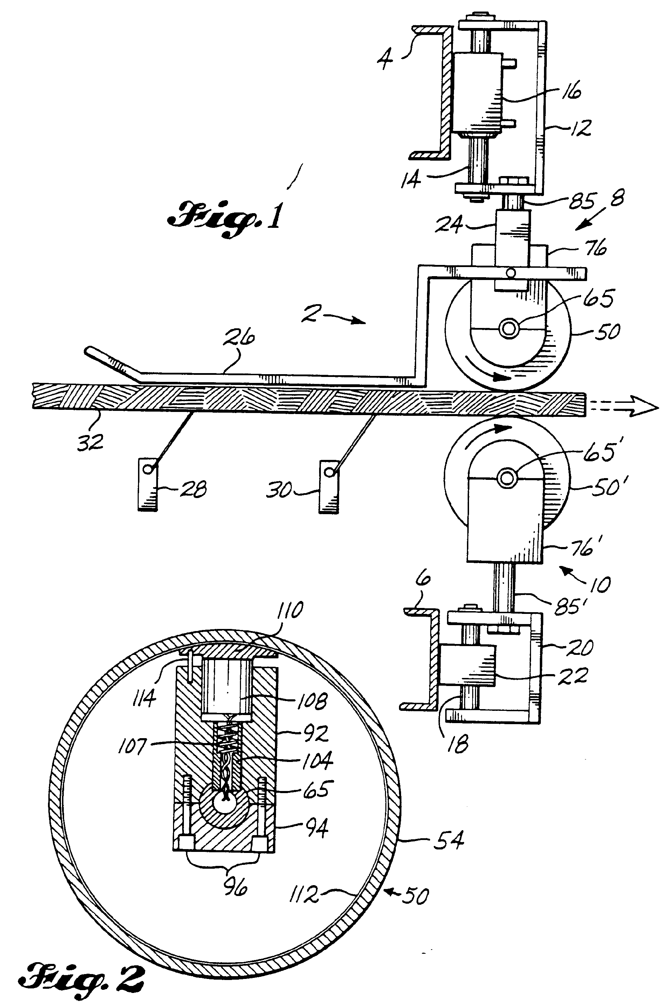

The apparatus used with the present method is conventional and, for the most part, can be assembled from components which are commercially available. Figure 1 shows a typical measurement setup as it might be used in a panel manufacturing operation, such as a particleboard mill. The testing apparatus, generally shown at 2, has frame members 4, 6 on which are mounted respectively an upper transducer wheel assembly 8 and lower transducer wheel assembly 10. The upper assembly has a subframe 12 which is mounted to both ends of a piston rod 14 in a double acting cylinder 16. An upper yoke 24 is mounted to subframe 12 by a shouldered bolt or similar mounting means 85. Mounted within yoke 76 on axle 65 is a rolling transducer 50. The lower rolling transducer assembly 10 is constructed in somewhat similar fashion with a support rod 18 holding subframe 20. The support rod is journaled in a bushing 22 where, in this embodiment, it is free to have limited rotation but fixed so that it will not translate up or down. Mounting member 85', which may also be a shouldered bolt, holds the lower yoke assembly 76' on which the lower rolling transducer assembly 50' is mounted on axle 65'.

An arm 26 is mounted on upper yoke 24 to detect the presence of overlapped panels which might damage the instrument. The shorter end of arm 26 is connected to a sensor mechanism, not shown, which will cause upper transducer assembly 8 to be withdrawn if overlapped panels are present.

The upper rolling transducer assembly 8 is normally raised out of the line of contact when no panels are passing. An approaching panel will contact presence/absence detectors 28, 30 and signal a control mechanism, not shown, to lower assembly 8 into contact with panel 32 after it is in position over lower transducer assembly 10. In similar fashion, when the trailing end of the panel clears switches 28, 30, transducer assembly 8 is raised before the panel has completely passed the rolling transducers. This eliminates impact damage to the upper transducer assembly where it might otherwise be hit by the leading edge of a panel or dropped as it leaves the trailing edge.

Two features of the invention are associated with the apparatus shown in Figure 1 but are not illustrated here. One of these is an optional panel thickness detector and the other is a panel temperature sensor. If a thickness detector is used, it may be either a contact type or a noncontact type, for example, a precision sonar surface location indicator.

Panel temperature may be measured or suitably estimated in a number of ways. It is the interior temperature of the panel which is important to the present invention. This may be closely estimated from surface temperature in most cases. For surface temperature detection a number of commercially available infrared measuring devices may be used. One which has proved satisfactory is the Optitherm® Series 12-8500, available from Pyrometer Instrument Company, Northvale, New Jersey. Another method of temperature estimation is based on a knowledge of the time that the panels have been out of the hot press upstream from the measuring position. The temperature within the press can be determined with acceptable accuracy as can the rate of heat loss as the panels move down the conveyor line. If the time between ejection from the press and measurement can be sufficiently well controlled, no temperature measurement instrumentation is required.

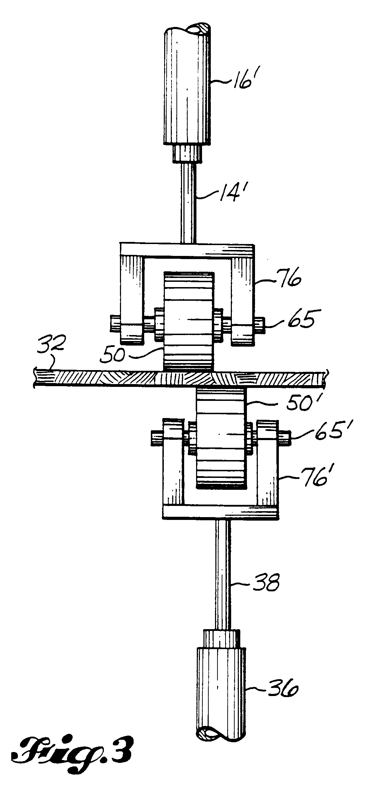

A rolling transducer assembly suitable for use with the present method is shown in Figure 2. This has a metallic rim 54 with face plates, not shown, mounted on each side and journaled about shaft 65. A transducer mount 92, 94 is connected within the rim and clamped in fixed position on shaft 65 by cap screws 96. Within the transducer mount is a sleeve 104 containing a spring or other resilient member 107 acting against a piezo electric transducer 108. Directly connected to the transducer is a rim contacting shoe 110 which floats on a thin oil film 112. A guide pin 114 may be necessary to help retain shoe 110 in its desired position. The lead wires from transducer 108 are brought out through an interior bore hole in shaft 65.

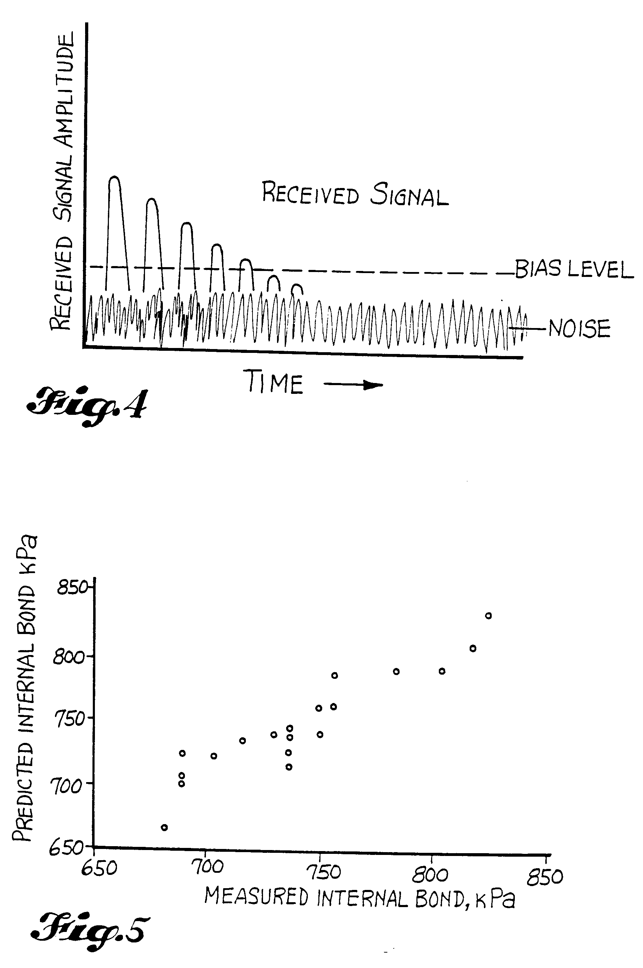

An alternative representation of the apparatus is seen in Figure 3, which is shown as an end-on elevation corresponding to the side elevation of Figure 1. The mounting of yoke 76 is simplified as shown here and it is located on piston rod 14' depending from cylinder 16'. In similar fashion, the lower rolling transducer assembly is shown with yoke 76' mounted on piston rod 38 of cylinder 36. It is acceptable for the two transducers to have contact points with the panel directly above each other. Stated otherwise the transducers may be located along a common axis. However, it is desirable that they be displaced somewhat in order to sample a larger portion of the panel being tested. The amount of displacement is a compromise between increased accuracy on the one hand and greater signal attenuation of the other. Any displacement between the two transducers may be either lateral, as shown in Figure 3, or it may be longitudinal. While in some cases the transducers may be located on the same side of the panel, it is preferable that they be located on opposite sides so that the pulse is transmitted through the entire thickness of the panel.

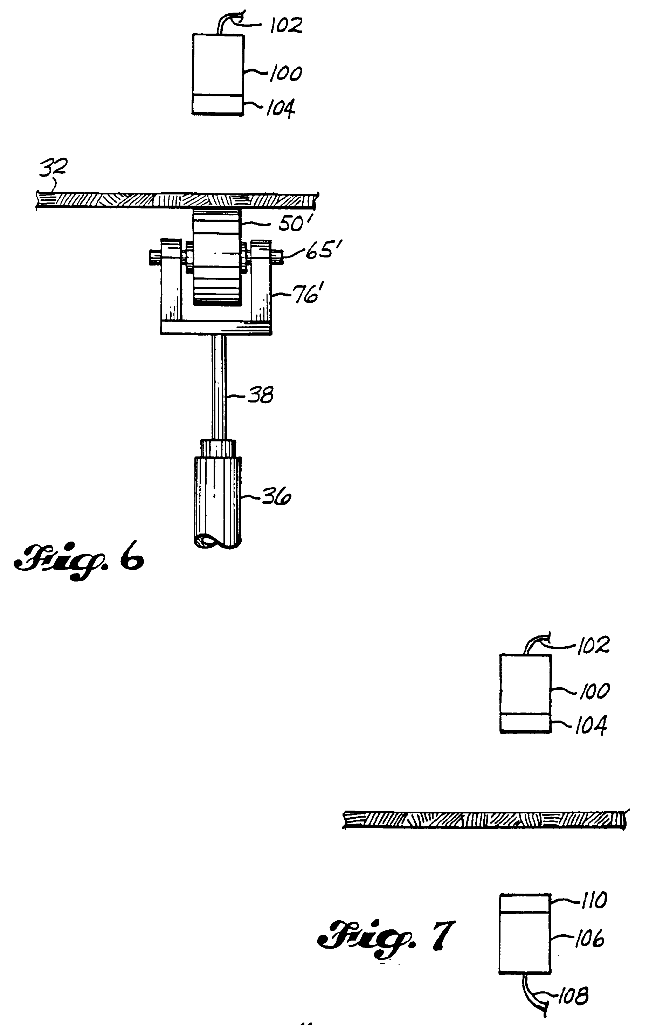

Suitable acoustic emission equipment for use with the present apparatus is available from a number of suppliers. One type of equipment that has proved suitable is an instrument designated Model 302, available from Acoustic Emission Technology Corporation, Sacramento, California. The acoustic driver directs an ultrasonic pulse against the panel. The received signal may be processed in one of two ways. In the first, the average RMS voltage produced in response to the transmitted pulse is measured. In the other, the ringdown count of the oscillations produced in response to the transmitted pulse is measured. Preferably, when this latter method is employed, the system is biased, as shown in Figure 4, so that only those pulses having a greater amplitude than the bias level are counted. In this way the bias level can be set above the ambient noise level of the system to achieve greater accuracy. A gating circuit may be used when RMS voltage received signals are being processed. This permits examination of signals for some discrete time period after each driver pulse and can reduce the contribution of spurious noise.

Figure 5 is a plot of 19 samples in which predicted internal bond from on-line measurement is compared with later laboratory tests of internal bond. The samples shown in this graph were measured between the temperature extremes of 40°C and 105°C on a 15,9 mm (5/8 inch) premium quality particleboard. The correlation, as shown by the statistical value $R^2$, was 0.85. When a similar set of measurements was attempted in which the ringdown voltage alone was considered, ignoring temperature, the value $R^2$ was only 0.4. In essence, under these conditions no correlation existed and there was a high probability that any perceived relationship between on-line and laboratory values was due entirely to chance. In the data collected for the graph shown in Figure 5, the panels were of sufficiently uniform thickness so that this component could be ignored in the algorithm used for calculation of internal bond strength. In this system the coefficients were as follows:

$$IB = -0.3 + 40.9(\text{ringdown voltage}) + 0.26(\text{temperature})$$

with internal bond expressed in lb/in² (1 lb/in² $\simeq$ 6.89x10³Pa) and temperature in °C. The values determined by this algorithm have been multiplied by a factor of 6.89 to convert them to kilopascals as illustrated on the graph.

While the best currently available technology would suggest the use of rolling transducers in physical contact with the panels being measured, this is not essential for achieving satisfactory results. Noncontacting ultrasonic transmitting and receiving transducers with sufficient power and senstitivy are presently available for use. A noncontact-type transmitting transducer may be used with either a contacting or noncontacting receiving transducer. Similarly, a contacting transmitter may be used with a contacting or noncontacting receiving transducer. Figures 6 and 7 are typical of these arrangements. Here an air coupled ultrasonic transmitter 100 is located above the panel 32 being measured. This is connected to an acoustic driver as described earlier by cable 102. Preferably a conventional impedance coupling device 104 is used to minimize signal loss at the air/transmitter interface. The rolling receiver 50 is also as described earlier.

In Figure 7 an air coupled receiving transducer 106 is also displaced from panel 32 an appropriate distance. This is connected by cable 108 to the receiver circuitry associated with the driver unit. The receiving transducer may be of any suitable type; e.g., one based on a piezoceramic crystal or one based on piezoelectric film such a polyvinyl fluoride.

As with the arrangement using two panel contacting rolling transducers, a frequency in the range of about 50-100 khz appears to give the best results.

The transmitting transducer 100 is preferably a piezoceramic crystal using conventional "tone burst" technology to drive the unit at its natural resonant frequency and increase output signal strength.

## Claims

1. A method for the on-line, nondestructive determination of a mechanical property of composite panel products (32) which comprises:

determining the thickness of the panel (32);

impinging an ultrasound pulse against the panel (32) through a first transducer (50, 100);

receiving the transmitted pulse at a second transducer (50', 106) spaced apart from the first transducer to provide a signal:

and determining the mechanical property in accordance with the signal and the thickness, **characterized in that**

said mechanical property is the internal bond strength of the panel product (32), and is determined by designating a thickness class for the measured panels (32), determining the temperature of the panel and expressing the signal received from the second transducer (50', 106) as an output voltage, entering the surface temperature and the output voltage into a linear equation of the form

IB = a + b(output voltage) + c(temperature)

where the coefficients are determined for the given thickness class; and solving the equation to determine the internal bond strength.

2. The method of claim 1 wherein the panel product (32) is a composite wood panel.

3. The method of claim 1 where the output of the second transducer (50', 106) is the average RMS voltage produced in response to the transmitted pulse.

4. The method of claim 1 where the output of the second transducer (50', 106) is a voltage analog of the ringdown count of the oscillations produced in response to the transmitted pulse.

5. The method of claim 4 in which the ringdown count is measured above a bias level set greater than the ambient noise level.

6. The method of claim 1 in which the second transducer (50', 106) is located on the opposite side of the panel (32) from the first transducer (50, 100).

7. The method of claim 6 in which the second transducer (50', 106) is linearly displaced either transversely or longitudinally from the point opposite the contact point of the first transducer (50, 100).

8. The method of claim 1 wherein the first transducer (50, 100) is spaced apart from the panel (32).

9. The method of claim 1 wherein the first and second transducers (50, 100, 50', 106) are spaced apart from the panel (32).

10. A method for the on-line, nondestructive determination of a mechanical property of composite panel products (32) which comprises:

determining the thickness of the panel (32);

impinging an ultrasound pulse against the panel (32) through a first transducer (50, 100);

receiving the transmitted pulse at a second transducer (50', 106) spaced apart from the first transducer to provide a signal;

and determining the mechanical property in accordance with the signal and the thickness, **characterized in that**

the said mechanical property is the internal bond strength of the panel product (32) and is determined by expressing the signal received from the second transducer (50', 106) as an output voltage, and determining the temperature of the panel (32), entering the thickness measured for each panel (32), the surface temperature, and the output signal voltage into an equation of the form

IB = a + b(output voltage) + c(temperature) + d(thickness) + e(thickness)$^2$;

and solving the equation to determine the internal bond strength.

11. The method of claim 10 wherein the panel product (32) is a composite wood panel.

12. The method of claim 10 where the output of the second transducer (50', 106) is the average RMS voltage produced in response to the transmitted pulse.

13. The method of claim 10 where the output of the second transducer (50', 106) is a voltage analog of the ringdown count of the oscillations produced in response to the transmitted pulse.

14. The method of claim 13 in which the ringdown count is measured above a bias level set greater than the ambient noise level.

15. The method of claim 10 in which the second transducer (50', 106) is located on the opposite side of the panel from the first transducer (50, 100).

16. The method of claim 15 in which the second transducer (50', 106) is linearly displaced either transversely or longitudinally from the point opposite the contact point of the first transducer (50, 100).

17. The method of claim 10 wherein the first transducer (50, 100) is spaced apart from the panel (32).

18. The method of claim 10 wherein the first and second transducers (50, 100, 50', 106) are spaced apart from the panel (32).

## Patentansprüche

1. Verfahren für eine zerstörungsfreie, on-line-Bestimmung einer mechanischen Eigenschaft von zusammengesetzten Plattenprodukten (32), das enthält:

die Bestimmung der Dicke der Platte (32);

das Aussenden einer Ultraschallschwingung gegen die Platte (32) durch einen ersten Energieumwandler (50, 100);

das Empfangen der übermittelten Schwingung an einem zweiten Energieumwandler (50', 106), der vom ersten Energieumwandler beabstandet ist, um ein Signal zu bilden;

und das Bestimmen der mechanischen Eigenschaft in Abhängigkeit vom Signal und der Dicke,

**dadurch gekennzeichnet,**

daß die mechanische Eigenschaft die innere Haftfestigkeit des Plattenproduktes (32) ist und bestimmt wird durch ein Festlegen einer Dickenklasse für die gemessenen Platten (32), ein Bestimmen der Temperatur der Platte und ein Festlegen des vom zweiten Energieumwandler (50', 106) empfangenen Signals als Ausgangsspannung, ein Einsetzen der Oberflächentemperatur und der Ausgangsspannung in eine lineare Gleichung der Form

IB = a + b (Ausgangsspannung) - c (Temperatur),

wobei die Koeffizienten für die vorgegebene Dickenklasse bestimmt werden, und durch das Lösen der Gleichung um die innere Haftfestigkeit zu bestimmen.

2. Verfahren nach Anspruch 1, wobei das Plattenprodukt (32) eine zusammengesetzte Holzplatte ist.

3. Verfahren nach Anspruch 1, wobei der Output des zweiten Energieumwandlers (50', 106) die mittlere RMS-Spannung ist, die gemäß der übertragenen Schwingung produziert wurde.

4. Verfahren nach Anspruch 1, wobei der Output des zweiten Energieumwandlers (50', 106) eine Spannung analog des Abklingens der Schwingungen ist, die gemäß der übertragenen Schwingung produziert wurden.

5. Verfahren nach Anspruch 4, wobei das Abklingen oberhalb eines Spannungsniveaus gemessen wird, das größer angesetzt wird als das Niveau des Umgebungsgeräusches.

6. Verfahren nach Anspruch 1, wobei der zweite Energieumwandler (50', 106) an der dem ersten Energieumwandler (50, 100) gegenüberliegenden Seite der Platte (32) angeordnet ist.

7. Verfahren nach Anspruch 6, wobei der zweite Energieumwandler (50', 106) linear entweder quer oder längs bezüglich des Punktes versetzt wurde, der dem Kontaktpunkt des ersten Energieumwandlers (50, 100) gegenüberliegt.

8. Verfahren nach Anspruch 1, wobei der erste Energieumwandler (50, 100) von der Platte (32) beabstandet ist.

9. Verfahren nach Anspruch 1, wobei der erste und der zweite Energieumwandler (50, 100, 50', 106) von der Platte (32) beabstandet sind.

10. Verfahren für die zerstörungsfreie, on-line-Bestimmung einer mechanischen Eigenschaft eines verbundenen Plattenproduktes (32), das umfaßt:

die Bestimmung der Dicke der Platte (32);

das Aussenden einer Ultraschallschwingung gegen die Platte (32) durch einen ersten Energieumwandler (50, 100);

das Empfangen der übermittelten Schwingung an einem zweiten Energieumwandler (50, 106), der vom ersten Energieumwandler beabstandet ist, um ein Signal zu bilden;

und das Besimmten der mechanischen Eigenschaft in Abhängigkeit vom Signal und der Dicke,

**dadurch gekennzeichnet,**

daß die mechanische Eigenschaft die innere Haftfestigkeit des Plattenproduktes (32) ist, und bestimmt wird durch das Festlegen des vom zweiten Energieumwandler (50', 106) empfangenen Signals als eine Ausgangsspannung, und das Bestimmen der Temperatur der Platte (32), das Einsetzen der gemessenen Dicke für jede Platte (32), die Oberflächentemperatur und die Ausgangsspannung in eine Gleichung der Form:

$$IB = a + b \text{ (Ausgangsspannung)} + c \text{ (Temperatur)} + d \text{ (Dicke)} + e \text{ (Dicke)}^2;$$

und das Lösen der Gleichung um die innere Haftfestigkeit zu bestimmen.

11. Verfahren nach Anspruch 10, wobei das Plattenprodukt (32) eine verbundene Holzplatte ist.

12. Verfahren nach Anspruch 10, wobei der Output des zweiten Energieumwandlers (50', 106) die mittlere RMS-Spannung ist, die gemäß der übertragenen Schwingung produziert wurde.

13. Verfahren nach Anspruch 10, wobei der Output des zweiten Energieumwandlers (50', 106) eine Spannung analog des Abklingens der Schwingungen ist, die gemäß der übertragenen Schwingung produziert wurden.

14. Verfahren nach Anspruch 13, wobei das Abklingen über einem Spannungsniveau gemessen wurde, das größer angesetzt wurde als das Niveau der Umgebungsgeräusche.

15. Verfahren nach Anspruch 10, wobei der zweite Energieumwandler (50', 106) an der dem ersten Energieumwandler (50, 100) gegenüberliegenden Seite der Platte angeordnet ist.

16. Verfahren nach Anspruch 15, wobei der zweite Umwandler (50', 106) in linearer Richtung, entweder quer oder längs gegenüber dem Punkt versetzt ist, der dem Kontaktpunkt des ersten Energieumwandlers (50, 100) gegenüberliegt.

17. Verfahren nach Anspruch 10, wobei der erste Energieumwandler (50, 100) von der Platte (32) beabstandet ist.

18. Verfahren nach Anspruch 10, wobei der erste und der zweite Energieumwandler (50, 100, 50', 106) von der Platte (32) beabstandet sind.

**Revendications**

1. Procédé de détermination en direct, non destructive, d'une propriété mécanique de produits en panneau composite (32), consistant :

à déterminer l'épaisseur du panneau (32) ;

à émettre une impulsion ultrasonore vers le panneau (32) par un premier transducteur (50, 100) ;

à recevoir l'impulsion émise par un second transducteur (50', 106) espacé du premier transducteur de manière à produire un signal ;

et à déterminer la propriété mécanique en fonction du signal et de l'épaisseur,

caractérisé en ce que

ladite propriété mécanique est la résistance à l'adhérence interne du produit en panneau (32) et elle est déterminée en spécifiant une plage d'épaisseur pour le panneau mesuré (32), en déterminant la température du panneau et en exprimant le signal reçu par le second transducteur (50′, 106) comme une tension de sortie, en introduisant la température superficielle et la tension de sortie dans une équation linéaire de la forme :

$$IB = a + b \text{ (tension de sortie)} + c \text{ (température)}$$

où les coefficients sont déterminés pour la plage d'épaisseur donnée ; et à résoudre l'équation pour déterminer la résistance à l'adhérence interne.

2. Procédé selon la revendication 1, dans lequel le produit en panneau (32) est un panneau en bois composite.

3. Procédé selon la revendication 1, dans lequel la sortie du second transducteur (50′, 106) est la tension moyenne efficace produite en réponse à l'impulsion emise.

4. Procédé selon la revendication 1, dans lequel la sortie du second transducteur (50′, 106) est une tension analogique d'amortissement décroissant des oscillations produites en réponse à l'impulsion émise.

5. Procédé selon la revendication 4, dans lequel le comptage décroissant est mesuré au-dessus d'un niveau de polarisation réglé au-dessus du niveau de bruit ambiant.

6. Procédé selon la revendication 1, dans lequel le second transducteur (50′, 106) est disposé sur le côté du panneau (32) opposé à celui du premier transducteur (50, 100).

7. Procédé selon la revendication 6, dans lequel le second transducteur (50′, 106) est décalé linéairement, transversalement ou longitudinalement, par rapport au point opposé au point de contact du premier transducteur (50, 100).

8. Procédé selon la revendication 1, dans lequel le premier transducteur (115, 100) est espacé du panneau (32).

9. Procédé selon la revendication 1, dans lequel le premier et le second transducteurs (50, 100, 50′, 106) sont espacés du panneau (32).

10. Procédé de détermination en direct, non destructive, d'une propriété mécanique de produits en panneau composite (32) , consistant,

à déterminer l'épaisseur du panneau (32) ;

à émettre une impulsion ultrasonore vers le panneau (32) au moyen d'un premier transducteur (50, 100) ;

à recevoir l'impulsion émise par un second transducteur (50′, 106) espacé du premier transducteur pour produire un signal ;

et à déterminer la propriété mécanique en fonction du signal et de l'épaisseur,

caractérisé en ce que

ladite propriété mécanique est la résistance en adhérence interne du produit en panneau (32) et elle est déterminée en exprimant le signal reçu, provenant du second transducteur, (50′, 106) sous la forme d'une tension de sortie et en déterminant la température du panneau (32), en introduisant l'épaisseur mesurée pour chaque panneau (32), la température superficielle et la tension du signal de sortie dans une équation de la forme :

$$IB = a + b \text{ (tension de sortie)} + c \text{ (température )} + b \text{ (épaisseur)} + e \text{ (épaisseur)}^2;$$

et en résolvant l'équation pour déterminer la résistance en adhérence interne.

11. Procédé selon la revendication 10, dans lequel le produit en panneau (32) est un panneau de bois composite.

12. Procédé selon la revendication 10, dans lequel la sortie du second transducteur (50′, 106) est la tension moyenne efficace produite en réponse à l'impulsion émise.

13. Procédé selon la revendication 10, dans lequel la sortie du second transducteur (50′, 106) est une tension analogique du comptage de décroissance des oscillations produites en réponse à l'impulsion émise.

14. Procédé selon la revendication 13, dans lequel le comptage décroissant est mesuré au-dessus d'un niveau de polarisation réglé au-dessus du niveau de bruit ambiant.

15. Procédé selon la revendication 10, dans lequel le second transducteur (50′, 106) est situé sur le côté du panneau opposé à celui du premier transducteur (50, 100).

16. Procédé selon la revendication 15, dans lequel le second transducteur (50′, 106) est décalé linéairement, transversalement ou longitudinalement, par rapport au point opposé au point de contact du premier transducteur (50, 100).

17. Procédé selon la revendication 10, dans lequel le premier transducteur (50, 100) est espacé du panneau (32).

18. Procédé selon la revendication 10, dans lequel le premier et le second transducteurs (50, 100, 50′, 106) sont espacés du panneau (32).

EP 0 322 446 B1

Fig. 1

Fig. 2

16'

14'

76

65

32

50

50'

65'

76'

38

36

*Fig.*3

*Fig.4*

*Fig.5*

102

100

104

32

50'

65'

76'

38

36

*Fig.* 6

102

100

104

110

106

*Fig.* 7

108

11